# FASCICULE DE BREVET EUROPEEN

(11) **EP 0 588 674 B1**
(45) Date de publication et mention de la délivrance du brevet: **23.04.1997**
(21) Numéro de dépôt: 93402040.5
(22) Date de dépôt: 11.08.1993
(51) Int. Cl.: C07C 227/18, C07C 229/24, C12P 13/20

(54) **Procédé de préparation de l'acide l-aspartique via l'aspartate d'ammonium**
Herstellung von Aspartinsäure von Ammonium-Aspartat
Preparation of L-aspartic acid via ammonium aspartate

(30) Priorité: 15.09.1992 FR 9210954
(43) Date de publication de la demande: 23.03.1994
(73) Titulaire: RHONE-POULENC CHIMIE, 92408 Courbevoie Cédex (FR)
(72) Inventeur: Brun, Daniel, F-69390 Charly (FR); Lahary, Pierre-Yves, F-69006 Lyon (FR); Thierry, Jean-François, F-69340 Francheville (FR)
(74) Mandataire: Dubruc, Philippe

(56) Documents cités:
- EP-A- 0 110 422
- GB-A- 2 108 128
- US-A- 3 933 586

## Description

La présente invention se rapporte à un procédé de préparation de l'acide L-aspartique.

Classiquement, l'acide L-aspartique est obtenu à partir de l'asparrtate d'ammonium qui lui-même est issu généralement d'un traitement enzymatique du fumarate d'ammonium. La précipitation de l'acide L-aspartique à partir de l'aspartate d'ammonium est effectuée à l'aide d'un acide, minéral ou organique, possédant un pKA inférieur à l'acidité correspondante de l'acide aspartique, soit inférieur à 3,65. Parmi les acides couramment employés, on peut notamment citer les acides minéraux, dont plus particulièrement l'acide sulfurique. L'acide maleique peut également être employé (EP-A-127 940) ; toutefois, on observe conjointement à la formation de l'acide L-aspartique attendu, la production de maléate d'ammonium, qu'il faut ensuite isomériser en fumarate d'ammonium, puis transformer en aspartate d'ammonium ; l'étape d'isomérisation entraine un surcroît de coût d'exploitation.

Il a également été proposé de préparer de l'acide aspartique par précipitation à partir d'un aspartate de métal alcalin (aspartate disodique) à l'aide d'acide fumarique (JP-B2-52 20 568).

La présente invention a pour objet un procédé de préparation d'acide L-aspartique par réaction d'aspartate d'ammonium et d'acide fumarique avec précipitation de l'acide L-aspartique, caractérisé en ce que la réaction de précipitation est réalisée à une température inférieure ou égale à 100°C, à partir d'un milieu hétérogène liquide-solide, l'acide fumarique étant mis en oeuvre dans un rapport molaire acide fumarique ajouté/aspartate d'ammonium présent compris entre environ 0,1 et 0,65, la concentration en eau du milieu de précipitation étant comprise entre environ 40 et 90% en poids, sans tenir compte de la quantité d'acide fumarique présent dans ledit milieu.

En fait cette aptitude de l'acide fumarique à précipiter efficacement l'acide aspartique à partir de l'aspartate d'ammonium en phase hétérogène est très surprenante.

Les valeurs des deux pKa de l'acide fumarique ne sont en effet pas favorables à la précipitation de l'acide L-aspartique. Leurs valeurs respectives de 3 et 4,4 sont du même ordre de grandeur que le pKa de l'acide aspartique.

En outre, l'acide fumarique, contrairement à l'acide maleique, présente une solubilité nettement plus réduite en milieu aqueux et à température ambiante. La réaction de précipitation est, donc, en présence d'acide fumarique à une température inférieure ou égale à 100°C, réalisée en milieu hétérogène. Toutefois, cette hétérogénéité a, contre toute attente, aucune incidence sur le bon déroulement de la réaction.

A la fin de la réaction, l'acide L-aspartique formé est isolé du milieu réactionnel, de préférence par filtration, lavé et séché. Selon un mode de réalisation particulier de l'invention, le filtrat contenant le fumarate d'ammonium est conservé.

Le rendement de la réaction en acide L- aspartique peut atteindre des valeurs comprises entre 70 et 95%.

La présente invention a pour second objet un procédé de préparation de l'acide L-aspartique comprenant, outre l'étape de précipitation de l'acide L-aspartique à l'acide fumarique, la formation préliminaire de l'aspartate d'ammonium à partir de fumarate d'ammonium.

Plus précisément la présente invention s'étend aux procédés comprenant en outre l'obtention au préalable de l'aspartate d'ammonium par traitement enzymatique du fumarate d'ammonium par des aspartases ou des microrganismes producteurs d'aspartases.

L'aptitude des aspartases à convertir le fumarate d'ammonium en aspartate est bien connue et de nombreuses méthodes pour produire l'aspartate par traitement enzymatique des aspartases avec du fumarate d'ammonium ont déjà été décrites dans la littérature. En conséquence, ces techniques ne seront pas rappelées ici.

Les microorganismes aptes à produire les aspartases incluent notamment les souches suivantes: Pseudomonas fluorescens, Escherichia coli, Aerobacter aerogenes, Bacterium succinium, Micrococcus sp, Bacillus subtilis et Serratia marcescens.

L'aspartate d'ammonium obtenu à l'issu du traitement enzymatique est de préférence isolé à la fin de la réaction en vu de l'étape de précipitation ultérieure à l'acide fumarique.

L'emploi de l'acide fumarique pour précipiter l'acide L-aspartique s'avère dans ce second cas particulièrement intéressant. En effet, le sous produit obtenu à l'issu de l'étape de précipitation c'est à dire le fumarate d'ammonium, peut avantageusement constituer une source en acide L-aspartique. Il peut être recyclé pour préparer de l'acide L-aspartique via la formation d'aspartate d'ammonium et permet donc de fonctionner en circuit fermé. La fabrication de sous produit est alors totalement inexistante.

En conséquence, selon un mode de réalisation préféré de l'invention, le milieu obtenu à l'issu de l'étape de précipitation de l'acide aspartique et débarrassé dudit acide, est utilisé à titre de source de fumarate d'ammonium pour préparer de l'aspartate d'ammonium.

Ce milieu peut être utilisé directement, sans traitement préalable quelconque, pour la préparation d'aspartate d'ammonium. Dans cette perspective les microorganismes aptes à produire l'aspartase y sont directement introduits avec, le cas échéant, réajustement de la stöechiométrie en ammoniaque.

Les exemples présentés ci-après, à titre non limitatif de la présente invention, mettent en évidence d'autres avantages du procédé revendiqué.

Dans ces exemples, un certain nombre de contrôles sont effectués:
La pureté de l'acide L-aspartique est contrôlée par potentiométrie ( dosage par la soude 1N).
La quantité d'acide fumarique n'ayant pas réagie est également dosée par chromatographie CLHP.

### Exemple 1 :

### Préparation de l'aspartate d'ammonium.

Dans un ballon de 2 l, muni d'une agitation magnétique, on introduit 300 g de NH4OH à 23,14 % en poids de NH3 et 800 g d'eau. On ajoute ensuite progressivement 543 g d'acide L-aspartique de façon à maintenir la température du milieu inférieure à 50°C. Après refroidissement, on obtient une solution homogène d'aspartate d'ammonium à 37,27 % P/P.

### Exemple 2 :

### Essai n° 1 de précipitation de l'acide aspartique

Dans un erlenmeyer de 100 ml, on charge 3,6 g d'acide fumarique avec 25 g d'eau. On ajoute ensuite 25 g de la solution d'aspartate d'ammonium à 37,27 % P/P (0,0621 mole) de l'exemple 1. La concentration en eau dans le milieu évaluée avant réaction, sans tenir compte de l'acide fumarique présent, **C**, est alors de 81,37 % P/P et le rapport molaire acide fumarique ajouté/aspartate d'ammonium présent, α, de 0,5.

Le milieu liquide-solide est agité à l'aide d'un agitateur magnétique pendant le temps **t** = 1 h à la température **T** = 20°.C La suspension obtenue est filtrée et l'acide L-aspartique récupérée. Après lavage et séchage on obtient 67 g de solide sec. Le rendement calculé en acide L-aspartique, **R**, est égal à 81,1 % molaire (nb de moles d'acide L-aspartique obtenues / nb de moles L-aspartate d'ammonium engagées) et le taux de transformation calculé en acide fumarique, **T**, est égal à 81,1 % (nb de moles d'acide L-aspartique obtenues / (2x(nb de moles d'acide fumarique engagées)).

La pureté de l'acide L-aspartique obtenu, déterminée par potentiométrie, **P** est de 101,1 %, et la teneur en acide fumarique déterminée par CLHP, **FA**, de 1,8%.

### Exemple 3 :

### Influence de la température sur la précipitation de l'acide L-aspartique

L'essai 2, présenté ci-après, est réalisé dans des conditions opératoires analogues à celles décrites dans l'exemple 2 (essai 1) mais en portant le mélange réactionnel au reflux à 100°C.

L'essai 3 est effectué selon le protocole suivant : 99,3 g de la solution d'aspartate d'ammonium à 37,27 % P/P (0.2467 mole) de l'exemple 1, 90,7 g d'eau et 14,3 g d'acide fumarique (0.1233 mole) sont chargés dans un réacteur en inox de 300 ml muni d'un hublot de verre permettant d'observer le milieu réactionnel. Le chauffage du réacteur est électrique et l'agitation est assurée par une vis d'Archimède. Après purge à l'azote, la consigne de température est portée à 135°C et l'agitation démarrée. Après 15 minutes, la température du milieu réactionnel atteint 135°C sous pression autogène ; le milieu réactionnel est alors liquide et homogène. La température est maintenue à 135 °C pendant 10 minutes. On laisse refroidir ensuite le réacteur et l'on procède ensuite à la vidange quand la température du milieu réactionnel atteint 88°C.

Les résultats **R,T,P** et **FA** correspondants sont présentés dans le tableau I çi-après.

**TABLEAU I**

| ESSAI | T°C | C% P/P | α | t | R% | T% | P% | FA% |
|---|---|---|---|---|---|---|---|---|
| 1 | 20 | 81,37 | 0,5 | 1 h. | 81,1 | 81,1 | 101,1 | 1,8 |
| 2 | 100 | 81,37 | 0,5 | 1,5 h. | 72 | 72 | 100,2 | 0,65 |
| 3 | 135 | 80,53 | 0,5 | 10 min. | 62,5 | 62,5 | 100,7 | 1,1 |

Les résultats révèlent que c'est pour les plus faibles températures que l'on obtient de meilleurs rendements pour des teneurs en acides fumariques satisfaisantes.

### Exemple 4

### Influence de la concentration en eau sur la précipitation de l'acide aspartique.

Elle a été étudiée à deux valeurs de températures 20°C et 100°C. Le tableau II ci-après rend compte des résultats correspondants.

Les essais 4, et 5 ont été effectués conformément au mode opératoire présenté en exemple 2 (essai 1).

En ce qui concerne l'essai 6, il a été effectué comme suit: 25 g de la solution d'aspartate d'ammonium à 37,27 % P/P (0.0621mole) de l'exemple 1 sont évaporés à 35°C sous vide de 10 mmHg en évaporateur rotatif. On obtient alors 19,1 g de solution d'aspartate d'ammonium à 48,77 % P/P. Cette solution est chargée dans un erlenmeyer de 50 ml. L'agitation est assurée à l'aide d'un agitateur magnétique. On ajoute ensuite 3,6 g d'acide fumarique. Le milieu réactionel est alors porté à reflux (température **T** = 100°C).

**TABLEAU II**

| ESSAI | C% P/P | α | T°C | t | R% | T% | FA% |
|---|---|---|---|---|---|---|---|
| 4 | 81,37 | 0,3 | 20 | 1 h. | 51,6 | 86 | 1,4 |
| 5 | 95,01 | 0,3 | 20 | 1 h. | 28,1 | 46,8 | 0,7 |
| 6 | 51,23 | 0,5 | 100 | 1 h. | 71 | 71 | 4,5 |
| 2 | 81,37 | 0,5 | 100 | 1,5 h. | 72 | 72 | 0,65 |

A très forte teneur en eau, les rendements diminuent de façon très significative.

### Exemple 5:

### Influence du paramètre α sur la réaction de précipitation.

De manière identique à l'exemple précédent, l'influence du paramètre α a été étudiée à deux valeurs de température, 20°C et 100°C.

L'essai 7 est réalisé selon le même mode opératoire que l'exemple 2 (essai 1).

Les essais 8 et 9 sont réalisés selon le même mode opératoire que l'essai 2, en engageant 0,0621 mole d'aspartate d'ammonium. L'essai 10 est effectué à 140°C selon le mode opératoire décrit pour l'essai 3 en engageant de même 0, 2467 mole d'aspartate d'ammonium.

Les résultats sont présentés dans le tableau III ci-après.

**TABLEAU III**

| ESSAI | α | C% P/P | T°C | t | R% | T% | FA% |
|---|---|---|---|---|---|---|---|
| 4 | 0,3 | 81,37 | 20 | 1 h. | 51,6 | 86 | 1,4 |
| 1 | 0,5 | 81,37 | 20 | 1 h. | 81,1 | 81,1 | 1,8 |
| 7 | 0,6 | 81,37 | 20 | 1 h. | 87,4 | 72,9 | 3,5 |
| 2 | 0,5 | 81,37 | 100 | 1,5 h. | 72 | 72 | 0,65 |
| 8 | 0,8 | 81,37 | 100 | 1,5 h. | 88 | 55 | 10 |
| 9 | 1 | 81,37 | 100 | 3 h | 87 | 43,5 | 22 |
| 10 | 1 | 80,53 | 140 | 10 min. | 79 | 39,5 | 25 |

On note que pour une valeur de α supérieure à 0,8, le taux en acide fumarique résiduel devient très élevé.

### Exemple 6 :

### Précipitation de l'acide L-aspartique en présence de fumarate d'ammonium.

Dans un erlenmeyer de 100 ml, on charge 1,79 g d'acide fumarique avec 25 g d'eau. On ajoute ensuite 24,8 g de la solution d'aspartate d'ammonium à 37,27 % P/P (0,0617 mole) de l'exemple 1. Le milieu liquide - solide est alors agité pendant 1h à la température de 20°C. Après filtration, lavage et séchage on obtient 3,49 g d'acide L-aspartique.

40, 22 g des eaux mères sont récupérées après filtration et chargées dans un erlenmeyer de 100 ml. On ajoute 8,78 g de la solution d'aspartate d'ammonium à 37,27 % de l'exemple 1 et ensuite 1,49 g d'acide fumarique. Le milieu est alors agité pendant 1h à 20°C. Après filtration, lavage et séchage, on obtient 3,05 g d'acide L-aspartique. 37,59 g des eaux mères sont récupérées après filtration et chargées dans un erlenmeyer de 100 ml. On ajoute 7,3 g de la solution d'aspartate d'ammonium à 37,27 % P/P de l'exemple 1 et ensuite 1,18 g d'acide fumarique. Le milieu est alors agité pendant 1 h à 20°C Après filtration, lavage et séchage, on obtient 2,08 g d'acide L-aspartique.

Pour les trois étapes, on calcule de surplus le rapport molaire, β, acide fumarique/aspartate d'ammonium présent au début de réaction de l'étape considérée, ainsi que le rendement cumulé sur les étapes de recyclage, RC (nombre de moles d'acide L-aspartique obtenues jusqu'à l'étape considérée/nombre de moles d'aspartate d'ammonium engagées jusqu'à l'étape considérée).

Les conditions opératoires α et β ainsi que les résultats R, RC et FA sont donnés dans le tableau IV ci-après.

**TABLEAU IV**

| étape | α | β | R% | RC% | FA% |
|---|---|---|---|---|---|
| 1 | 0,25 | 0,25 | 42,6 | 42,6 | 0,6 |
| 2 | 0,25 | 0,294 | 44,6 | 61,2 | 0,8 |
| 3 | 0,25 | 0,321 | 38,4 | 67,2 | 1,5 |

## Revendications

1. Procédé de préparation d'acide L-aspartique par réaction d'aspartate d'ammonium et d'acide fumarique avec précipitation de l'acide L-aspartique, caractérisé en ce que la réaction de précipitation est réalisée à une température inférieure ou égale à 100°C, à partir d'un milieu hétérogène liquide-solide, l'acide fumarique étant mis en oeuvre dans un rapport molaire acide fumarique ajouté/aspartate d'ammonium présent compris entre environ 0,1 et 0,65, la concentration en eau du milieu de précipitation étant comprise entre environ 40 et 90% en poids, sans tenir compte de la quantité d'acide fumarique présent dans ledit milieu.

2. Procédé selon la revendication 1), caractérisé en ce que l'acide L-aspartique obtenu est isolé du milieu réactionnel contenant du fumarate d'ammonium.

3. Procédé selon la revendication 2), caractérisé en ce que l'acide aspartique est isolé par filtration.

4. Procédé selon la revendication 1) ou 2), caractérisé en ce que l'aspartate d'ammonium mis en oeuvre est obtenu au préalable par traitement enzymatique de fumarate d'ammonium par des aspartases ou de microorganismes producteurs d'aspartases.

5. Procédé selon la revendication 2) ou 3), caractérisé en ce que le milieu obtenu après isolement de l'acide L-aspartique est utilisé à titre de source de fumarate d'ammonium pour préparer l'aspartate d'ammonium.

## Claims

1. Process for the preparation of L-aspartic acid by reaction of ammonium aspartate and fumaric acid with precipitation of the L-aspartic acid, characterized in that the precipitation reaction is carried out at a temperature of less than or equal to 100°C, from a heterogeneous solid/liquid mixture, the fumaric acid being employed in an added fumaric acid/ammonium aspartate present molar ratio of between approximately 0.1 and 0.65 and the water concentration of the precipitation mixture being between approximately 40 and 90 % by weight, without taking into account the amount of fumaric acid present in the said mixture.

2. Process according to claim 1, characterized in that the L-aspartic acid obtained is isolated from the reaction mixture containing ammonium fumarate.

3. Process according to claim 2, characterized in that the aspartic acid is isolated by filtration.

4. Process according to claim 1 or 2, characterized in that the ammonium aspartate employed is obtained beforehand by enzymatic treatment of ammonium fumarate with aspartases or microorganisms which produce aspartases.

5. Process according to claim 2 or 3, characterized in that the mixture obtained after isolation of the L-aspartic acid is used as source of ammonium fumarate for preparing ammonium aspartate.

## Patentansprüche

1. Verfahren zur Herstellung von L-Asparaginsäure durch Umsetzung von Ammoniumaspartat mit Fumarsäure unter Ausfällung der L-Asparaginsäure, dadurch **gekennzeichnet,** daß man die Fällungsreaktion bei einer Temperatur von kleiner oder gleich 100°C aus einem heterogenen Flüssig-Fest-Medium durchführt, wobei die Fumarsäure in einem Molverhältnis zugesetzte Fumarsäure/vorhandenes Ammoniumaspartat zwischen etwa 0,1 und 0,65 eingesetzt wird, die Wasserkonzentration des Fällungsmediums zwischen etwa 40 und 90 Gew. % liegt, wobei die in dem Reaktionsmedium vorhandene Fumarsäure nicht berücksichtigt wird.

2. Verfahren nach Anspruch 1, dadurch **gekennzeichnet,** daß man die so erhaltene L-Asparaginsäure aus dem Anmoniumfumarat enthaltenden Reaktionsmedium isoliert.

3. Verfahren nach Anspruch 2, dadurch **gekennzeichnet,** daß man die Asparaginsäure durch Filtration isoliert.

4. Verfahren nach Anspruch 1 oder 2, dadurch **gekennzeichnet,** daß man das eingesetzte Anmoniumaspartat durch vorherige enzymatische Behandlung von Anmoniumfumarat mit Aspartasen oder Aspartasen produzierenden Mikroorganismen erhält.

5. Verfahren nach Anspruch 2 oder 3, dadurch **gekennzeichnet,** daß man das nach der Isolierung der L-Asparaginsäure erhaltene Reaktionsmedium als Quelle für Ammoniumfumarat zur Herstellung von Ammoniumaspartat verwendet.
